# EUROPEAN PATENT APPLICATION

(11) **EP 2 409 688 A1**
(43) Date of publication of application: **25.01.2012**
(21) Application number: 10753428.1
(22) Date of filing: 08.03.2010
(51) Int. Cl.: A61K 9/20, A61K 47/04, A61K 47/10, A61K 47/26

(54) **ORALLY DISINTEGRATING TABLET**

(30) Priority: 16.03.2009 JP 2009063148
(71) Applicant: NIPRO CORPORATION, Kita-ku Osaka-shi, Osaka 531-8510 (JP)
(72) Inventor: HOASHI, Yohei, Osaka-shi Osaka 531-8510 (JP); TAKEUCHI, Hirofumi, Gifu-shi Gifu 502-0006 (JP)
(74) Representative: Beckmann, Claus
(86) International application number: PCT/JP2010/053761
(87) International publication number: WO 2010/106936

(57) **Abstract**

It is an object to provide an orally disintegrating tablet produced by dry granulation and compression, having a hardness of 40 N or more, a disintegration time of 30 seconds or shorter, a friability of 0.1% or less, and an excellent feeling upon ingestion, that is capable of disintegrating with a small amount of water, having a rapid disintegration time, and being maintained stably in a tablet form, which could not been achieved by conventional procedures.

Disclosed is an orally disintegrating tablet produced by dry granulation which contains a medicinal ingredient with silica, and sugar alcohol or/and sugar.

## Description

### Technical Field

The present invention relates to an orally disintegrating tablet that can be produced using dry granulation.

### Background Art

Orally disintegrating tablets rapidly disintegrate in the oral cavity when they are taken so as to facilitate their ingestion, and therefore are to be particularly preferably prescribed to people who have low swallowing ability, in particular, children, elderly people, and the like, due to their easiness and convenience in ingestion.

It is necessary that orally disintegrating tablets are maintained in a tablet form stably during from production till packaging, transport, and storage while ensuring that the tablets have the ability to rapidly disintegrate in the oral cavity. Therefore, specifically, the so-called orally disintegrating tablets are usually at least required to disintegrate within 30 seconds in the oral cavity and have a tablet hardness of 40 N or more.

Embodiments of orally disintegrating tablets known at this time include tablets that will be described below.
Patent Document 1 discloses an orally disintegrating tablet containing sugar or sugar alcohol having an average particle size of 30 µm to 300 µm. In the Examples in Patent Document 1, orally disintegrating tablets obtained by wet granulation or direct compression are disclosed, and the characteristics of the obtained tablets are also shown in Table 1.
The orally disintegrating tablets disclosed in Patent Document 1 have inadequate tablet hardness as shown in Table 1. Since the case of a tablet containing a water-labile medicinal substance cannot be produced using wet granulation, then dry granulation or direct compression is used. Dry granulation is mentioned in the specification, but is not specifically recognized in the Examples and the like. Direct compression is disclosed in Examples 13 to 15, however, it takes long time of 38 to 67 seconds to distintegrate in the oral carvity for normal adults, and even more time required can be predicted for disintegration upon ingestion by children or elderly people, which are not preferred.

As mentioned in the paragraph (0004), dry granulation can be used as one of the methods for producing a tablet containing a water-labile medicinal substance.
Patent Document 2 discloses an orally disintegrating tablet produced using dry granulation, with starch and crystalline cellulose and/or powdered cellulose. The orally disintegrating tablet produced using dry granulation is clearly described in a specific manner in Example 3 in Patent Document 2, and it is also described there that the obtained tablet has a hardness of 72 N and a disintegration time of 13 seconds in the oral cavity.

As mentioned in the paragraph (0005), an orally disintegrating tablet can be produced by dry granulation with crystalline cellulose and/or powdered cellulose to achieve adequately a high hardness and a reduced disintegration time in the oral cavity for the orally disintegrating tablet. However, Non-Patent Document 1 and the like have shown that the tablet with the aforementioned celluloses requires a large amount of water to disintegrate. It can be seen from Fig. 11 of Non-Patent Document 1 that powdered cellulose and low substituted hydroxypropyl cellulose are extremely highly hygroscopic, and such tablets require a large amount of water to disintegrate.

Meanwhile, orally disintegrating tablets are to be particularly preferably prescribed to children and elderly people, who secrete less saliva in the oral cavity than normal adults, which may cause inadequate disintegration of tablet or difficulty in swallowing. Therefore, there is a demand for formulation of an orally disintegrating tablet that capable of disintegrating with a smaller amount of water.

Patent Document 3 discloses producing an orally disintegrating tablet with improved mechanical strength by merely previously compounding a small part of sugars serving as an excipient in the tablet with silica and performing mixing. Orally disintegrating tablets produced in such a manner are high in sugar content and can provide a favorable feeling upon ingestion. However, in such a manner, the poor flowability of powders varies the loaded amount of powders in a mill, which is problematic. Moreover, the tablet has adequate hardness but inadequate friability, and there is concern that cracking or chipping may occur during production, packaging, transport, and the like.

### Prior Art Documents

### Patent Documents

Patent Document 1: Japanese Laid-Open Patent Publication No. 2001-058944
Patent Document 2: Japanese Laid-Open Patent Publication No. 2007-332074
Patent Document 3: Japanese Laid-Open Patent Publication No. 2006-248922

### Non-Patent Document

Non-Patent Document 1: Yakuzaigaku (Journal of Pharmaceutical Science and Technology, Japan) Vol. 66, No. 6 (2006) 473-481

### Summary of Invention

### Problems to be Solved by the Invention

It is an object of the present invention to provide an orally disintegrating tablet produced by dry granulation and compression, having a hardness of 40 N or more, a disintegration time of 30 seconds or shorter, a friability of 0.1% or less, and an excellent feeling upon ingestion, that is capable of disintegrating with a small amount of water, having a rapid disintegration time, and being maintained stably in a tablet form, which could not be achieved by conventional procedures.

### Means for Solving the Problems

The inventors of the present invention have conducted in-depth studies and have made it possible to produce a desired orally disintegrating tablet having the following features, which are different from conventional procedures.
(1) An orally disintegrating tablet produced by dry granulation, which contains a medicinal ingredient with silica, and sugar alcohol or/and sugar.
(2) The orally disintegrating tablet of (1), wherein the medicinal ingredient is water-labile.
(3) The orally disintegrating tablet of (1), wherein the sugar alcohol is mannitol, erythritol, xylitol, maltitol, sorbitol.
(4) The orally disintegrating tablet of (1), wherein the sugar is lactose, sucrose, glucose, trehalose.

Hereinafter, the present invention will now be described in detail.

"Dry granulation" as used herein is a general term for granulation methods that use no liquid component during granulation.
Examples of the dry granulation methods that can be used in the present invention include methods employing a roller compactor, a Pharmapaktor, a Chilsonator, a rotary press, or the like.

A medicinal ingredient that can used in the present invention may be in any form, such as solid form, crystalline form, oil form, or solution form, and one or two or more medicinal ingredients selected from, for example, antipyretic antiinflammatory analgesics, analeptic and health-care drugs, psychotropic drugs, antidepressants, antianxiety drugs, sedative-hypnotics, antispasmodics, central nervous system agents, cerebral metabolism improving agents, cerebral blood flow improving agents, antiepileptic drugs, sympathomimetic agents, gastrointestinal drugs, antacids, antiulcer agents, antitussives/expectorants, antiemetics, respiratory stimulants, bronchodilators, antiallergic drugs, antihistamines, agents for dental and oral use, cardiotonics, antiarrhythmic agents, diuretics, hypotensive agents, vasoconstrictors, coronary vasodilators, peripheral vasodilators, anticoagulants, agents for hyperlipidemia, cholagogues, antibiotics, chemotherapeutic agents, agents for diabetes, agents for osteoporosis, antirheumatic drugs, skeletal muscle relaxants, spasmolytics, hormone preparations, alkaloid narcotics, sulfa drugs, drugs for treatment of gout, and antineoplastic drugs can be used.

Examples of the antipyretic antiinflammatory analgesics include acetaminophen, aspirin, ibuprofen, ethenzamide, diphenhydramine hydrochloride, dl-clalorpheniramine maleate, diclofenac sodium, dihydrocodeine phosphate, salicylamide, aminopyrine, noscapine, methylephedrine hydrochloride, phenylpropanolamine hydrochloride, serrapeptase, lysozyme hydrochloride, tolfenamic acid, mefenamic acid, flufenamic acid, ketoprofen, indomethacin, bucolome, pentazocine, caffeine, and anhydrous caffeine. Examples of the analeptic and health-care drugs include vitamins such as vitamin A, vitamin B1 (dibenzoyl thiamine, fursultiamine hydrochloride, and the like), vitamin B2 (riboflavin butyrate and the like), vitamin B6 (pyridoxine hydrochloride and the like), vitamin B12 (hydroxocobalamin acetate, cyanocobalamin, and the like), vitamin C (ascorbic acid, sodium L-ascorbate, and the like), vitamin D, and vitamin E (d-α-tocopherol acetate and the like); minerals such as calcium, magnesium, and iron; proteins; amino acids; oligosaccharides; and herbal medicines. Examples of the psychotropic drugs include chlorpromazine and reserpine. Examples of the antidepressants include amphetamine, imipramine, and maprotiline hydrochloride. Examples of the antianxiety drugs include diazepam, alprazolam, and chlordiazepoxide. Examples of the sedative-hypnotics include estazolam, diazepam, nitrazepam, perlapine, and phenobarbital sodium. Examples of the antispasmodics include scopolamine hydrobromide, diphenhydramine hydrochloride, and papaverine hydrochloride. Examples of the central nervous system agents include citicoline. Examples of the cerebral metabolism improving agents include meclofenoxate hydrochloride. Examples of the cerebral blood flow improving agents include vinpocetine. Examples of the antiepileptic drugs include phenytoin and carbamazepine. Examplse of the sympathomimetic agents include isoproterenol hydrochloride. Examples of the gastrointestinal drugs include stomachics and digestants such as diastase, saccharated pepsin, *Scopolia* extract, cellulase AP3, lipase AP, and cinnamon oil; and intestinal drugs such as berberine chloride, antibiotics-resistant lactic acid bacteria, and bifidobacteria. Examples of the antacids include magnesium carbonate, sodium bicarbonate, magnesium aluminometasilicate, synthetic hydrotalcite, precipitated calcium carbonate, and magnesium oxide. Examples of the antiulcer agents include lansoprazole, omeprazole, rabeprazole, cimetidine, famotidine, and ranitidine hydrochloride. Examples of the antitussives/expectorants include cloperastine hydrochloride, dextromethorphan hydrobromide, theophylline, potassium guaiacolsulfonate, guaifenesin, and codeine phosphate. Examples of the antiemetics include difenidol hydrochloride and metoclopramide. Examples of the respiratory stimulants include levallorphan tartrate. Examples of the bronchodilators include theophylline and salbutamol sulfate. Examples of the antiallergic drugs include amlexanox and seratrodast. Examples of the antihistamines include diphenhydramine hydrochloride, promethazine, isothipendyl hydrochloride, and dl-chlorpheniramine maleate. Examples of the agents for dental and oral use include oxytetracycline, triamcinolone acetonide, chlorhexidine hydrochloride, and lidocaine. Examples of the cardiotonics include digoxin and caffeine. Examples of the antiarrhythmic agents include procainamide hydrochloride, propranolol hydrochloride, and pindolol. Examples of the diuretics include furosemide, isosorbide, and hydrochlorothiazide. Examples of the hypotensive agents include captopril, delapril hydrochloride, hydralazine hydrochloride, labetalol hydrochloride, manidipine hydrochloride, candesartan cilexetil, methyldopa, and perindopril erbumine. Examples of the vasoconstrictors include phenylephrine hydrochloride. Examples of the coronary vasodilators include carbocromen hydrochloride, molsidomine, and verapamil hydrochloride. Examples of the peripheral vasodilators include cinnarizine. Examples of the anticoagulants include dicumarol. Examples of the agents for hyperlipidemia include cerivastatin sodium, simvastatin, pravastatin sodium, and atorvastatin calcium hydrate. Examples of the cholagogues include dehydrocholic acid and trepibutone. Examples of the antibiotics include cephem antibiotics such as cephalexin, amoxicillin, cefaclor, pivmecillinam hydrochloride, cefotiam hexetil hydrochloride, cefadroxil, cefixime, cefditoren pivoxil, cefteram pivoxil, and cefpodoxime proxetil; synthetic antimicrobials such as ampicillin, cyclacillin, nalidixic acid, and enoxacin; monobactam antibiotics such as carumonam sodium; penem antibiotics; and carbapenem antibiotics. Examples of the chemotherapeutic agents include sulfamethizole. Examples of the agents for diabetes include tolbutamide, voglibose, pioglitazone hydrochloride, glibenclamide, and troglitazone. Examples of the agents for osteoporosis include ipriflavone. Examples of the skeletal muscle relaxants include methocarbamol. Examples of the spasmolytics include meclizine hydrochloride and dimenhydrinate. Examples of the antirheumatic drugs include methotrexate and bucillamine. Examples of the hormone preparations include liothyronine sodium, dexamethasone sodium phosphate, prednisolone, oxendolone, and leuprorelin acetate. Examples of the alkaloid narcotics include opium, morphine hydrochloride, ipecac, oxycodone hydrochloride, opium alkaloid hydrochloride and cocaine hydrochloride. Examples of the sulfa drugs include sulfisomidine and sulfamethizole. Examples of the drugs for treatment of gout include allopurinol and colchicine. Examples of the antineoplastic drugs include 5-fluorouracil, uracil, and mitomycin. The active ingredient may be diluted with a diluent or the like commonly used in the field of medicine, food, etc., and may be treated to mask the bitter taste of its own.

Examples of medicinal ingredients that can be preferably used in the present invention are water-labile substances.
Specific examples of such medicinal ingredients include methylmethioninesulfonium chloride; amino acids (aspartic acid, cysteine, and the like); various vitamins (vitamin B1, vitamin B2, vitamin B6, vitamin B12, vitamin C, nicotinamide, vitamine P, derivatives of these vitamins, and the like) and enzymes (starch digesting enzymes, protein digesting enzymes, fat digesting enzymes, cellulolytic enzymes, and the like); and herbal medicines (garlic, oxoamidine, and powdered or dried extracts of *Astragalus* root, *Eleutherococcus senticosus,* hop (*Humulus lupulus*), *Coix* seed, *Swertia* herb, pilose antler (Cervi Parvum Cornu), *Glycyrrhiza, Platycodon* root, cinnamon bark, *Asiasarum* root, peony root, *Atractylodes lancea* rhizome, ginger, ginseng root, and the like).

Examples of the sugar alcohol that can be used in the present invention include mannitol, erythritol, xylitol, maltitol, and sorbitol.

Examples of the sugar that can be used in the present invention include lactose, sucrose, glucose, and trehalose.

Additives disclosed below can be further added to an orally disintegrating tablet provided according to the present invention, as long as the tablet exerts the effects of the invention.
Examples of the additives that can be used in the present invention include binders, lubricants, disintegrants, pH adjusting agents, fluidizers, surfactants, coloring agents, sweeteners, and coating agents.

Examples of the binders that can be used in the present invention includes ethyl acrylate-methyl methacrylate copolymer, aminoalkyl methacrylate copolymer RS, aminoalkyl methacrylate copolymer E, sodium alginate, ethyl cellulose, carrageenan, carboxyvinyl polymer, carboxy methyl ethyl cellulose, agar, copolyvidone, purified shellac, dextrin, hydroxyethyl cellulose, hydroxyethyl methyl cellulose, hydroxypropyl starch, hydroxypropyl cellulose, vinylpyrrolidone-vinyl acetate copolymer, hypromellose, partially pregelatinized starch, pullulan, pectin, polyvinyl alcohol-polyethylene glycol graft copolymer, povidone, polyvinyl alcohol, methacrylic acid copolymer L, methacrylic acid copolymer LD, methacrylic acid copolymer S, and methylcellulose.

Examples of the lubricants that can be used in the present invention include carmellose calcium, carmellose sodium, glycerin, glycerin fatty acid ester, wheat starch, sucrose fatty acid ester, stearyl alcohol, stearic acid, cetanol, gelatin, corn starch, potato starch, polyoxyethylene-polyoxypropylene glycol, polysorbate, macrogol, glyceryl monostearate, and sodium lauryl sulfate.

Examples of the disintegrants that can be used in the present invention include carmellose calcium, carboxymethyl starch sodium, croscarmellose sodium, crospovidone, cellulose or derivatives thereof, and starch or derivatives thereof.

Examples of the pH adjusting agents that can be used in the present invention include citric acid and its salts, phosphoric acid and its salts, carbonic acid and its salts, tartaric acid and its salts, fumaric acid and its salts, acetic acid and its salts, amino acids and its salts, succinic acid and its salts, and lactic acid and its salts.

Examples of the fluidizers that can be used in the present invention include light anhydrous silicic acid, hydrous silicon dioxide, titanium oxide, stearic acid, corn gel, and heavy anhydrous silicic acid.

Examples of the surfactants that can be used in the present invention include phospholipid, glycerin fatty acid ester, polyoxyethylene fatty acid ester, sorbitan fatty acid ester, polyethylene glycol fatty acid ester, polyoxyethylene hydrogenated castor oil, polyoxyethylene alkyl ether, sucrose fatty acid ester, sodium lauryl sulfate, polysorbates, sodium hydrogen phosphates, and potassium hydrogen phosphates.

Examples of the coloring agents that can be used in the present invention include iron sesquioxide, yellow iron sesquioxide, Food Yellow No. 5, Food Yellow No. 4, aluminum chelate, titanium oxide, and talc.

Examples of the sweeteners that can be used in the present invention include saccharin, aspartame, acesulfame potassium, thaumatin, and sucralose.

Examples of the coating agents that can be used in the present invention include hydroxypropyl cellulose, hydroxypropylmethylcellulose, methylcellulose, polyvinyl alcohol, polyvinylpyrrolidone-ethyl acrylate, methyl methacrylate copolymer dispersions, hydroxypropylmethylcellulose acetate succinate, and methacrylic acid copolymers.

According to the present invention, it is preferable that the content of the sugar alcohol or/and the sugar accounts for 50 to 90% of the content of the sugar alcohol or/and the sugar and the various additives excluding the medicinal ingredient. According to the present invention, it is also preferable that the silica and the sugar alcohol or/and the sugar form a composite particle.

There is no particular limitation to the tableting method for an orally disintegrating tablet provided according to the present invention, as long as the tablet exerts the effects of the invention. Examples of the tableting method that can be used in the present invention include a direct compression method, a dry indirect compression method, and a wet indirect compression method.

An orally disintegrating tablet provided by the present invention is shaped using, for example, a single-punch tableting machine, a rotary tableting press, or the like. Although there is no particular limitation to the shape of solid formulations according to the present invention, the tablet may be round, caplet, doughnut, oblong, and the like, may be multilayered, cored, and the like, and can be further coated with a coating agent. Optionally, the tablet may be provided with distinguishing characters, symbols, or marks, and may be provided with a dividing line so that the tablet can be divided.

### Effects of Invention

According to the present invention, it has became possible to provide an orally disintegrating tablet that is capable of disintegrating with a small amount of water, having a rapid disintegration time, and being maintained stably in a tablet form, which could not be achieved by conventional procedures.

### Best Mode for Carrying Out the Invention

Hereinafter, the best mode for carrying out the invention will be disclosed.

### Examples

### Example 1

Mannitol-silica composite particles were prepared by dissolving and dispersing 9 kg of mannitol and 6 kg of silica (Sylysia 350: Fuji Silysia Chemical Ltd.) in 85 kg of water and performing spray drying using a spray dryer (N-12: Ohkawara Kakohki Co., Ltd.).
Next, 1 part by weight of the mannitol-silica composite particles and 1 part by weight of crospovidone were added to and mixed with 8 parts by weight of mannitol. The mixture was dry granulated using a roller compactor (a model of TF-MINI: Freund Corporation), and then subjected to particle size regulation using a COMIL (a model of QC-1975: Powrex Corporation), and then subjected to a 75 µm sieve to eliminate fine powders, and the remaining was obtained as granules for tableting. Then, 1 part by weight of magnesium stearate was mixed with 99 parts by weight of the granules for tableting, and flat-faced bevel-edged tablets having a tablet diameter of 7.5 mm and a tablet weight of 150 mg were produced at a compression pressure of 9 kN.

### Example 2

Erythritol-silica composite particles were prepared by dissolving and dispersing 10 kg of erythritol and 5 kg of silica (Sylysia 350: Fuji Silysia Chemical Ltd.) in 50 kg of water and performing spray drying using a spray dryer (NB-12: Ohkawara Kakohki Co., Ltd.).
Next, 1 part by weight of the erythritol- silica composite particles and 1 part by weight of crospovidone were added to and mixed with 8 parts by weight of erythritol. The mixture was dry granulated using a roller compactor (a model of TF-MINI: Freund Corporation), and then subjected to particle size regulation using a COMIL (a model of QC-1975: Powrex Corporation), and then subjected to a 75 µm sieve to eliminate fine powders, and the remaining was obtained as granules for tableting. Then, 1 part by weight of magnesium stearate was mixed with 99 parts by weight of the granules for tableting, and flat-faced bevel-edged tablets having a tablet diameter of 7.5 mm and a tablet weight of 150 mg were produced at a compression pressure of 9 kN.

### Example 3

First, 0.8 kg of mannitol-silica composite particles and 0.8 kg of crospovidone were added to and mixed with 6.4 kg of mannitol. The mixture was dry granulated using a roller compactor (a model of WP160×60N: Turbo Kogyo Co., Ltd.), and then particle size regulation was performed using a roll granulator (GRN-T-54S: Nippon Granulator Co., Ltd.) to obtain granules for tableting. Then, 1 part by weight of magnesium stearate was mixed with 99 parts by weight of the granules after the particle size regulation, and flat-faced bevel-edged tablets having a tablet diameter of 8.0 mm and a tablet weight of 200 mg were produced at a compression pressure of 9 kN.

### Comparative Example 1

With 79 parts by weight of mannitol, 10 parts by weight of mannitol-silica composite particles, 10 parts by weight of crospovidone, and 1 part by weight of magnesium stearate were mixed, and flat-faced bevel-edged tablets having a tablet diameter of 8.0 mm and a tablet weight of 200 mg were produced at a compression pressure of 9 kN.

### Comparative Example 2

With 89 parts by weight of crystalline cellulose, 10 parts by weight of crospovidone and 1 part by weight of magnesium stearate were mixed, and flat-faced bevel-edged tablets having a tablet diameter of 8.0 mm and a tablet weight of 200 mg were produced at a compression pressure of 9 kN.

### Examination Example 1

The respective orally disintegrating tablets produced by the production methods described in Examples 1 to 3 and Comparative Example 1 were examined with regard to tablet hardness, disintegration time, and friability. The tablet hardness was determined using a tablet hardness tester (a model of 6D: Schleuniger). The tablet disintegration time was determined as follows: a tablet was introduced in the oral cavity of each of four subjects (healthy adult men and women), the time from the introduction until complete disintegration in the oral cavity of the tablet was measured, and an average was obtained from the measured times. The tablet friability was tested and determined in compliance with the friability testing method prescribed in the Japanese Pharmacopoeia 15th edition.
The results of determination were as shown in Table 1.

**[Table 1]**

| | Example 1 | Example 2 | Example 3 | Comparative Example 1 |
|---|---|---|---|---|
| Hardness | 45N | 60N | 56N | 58N |
| Friability | 0.083% | 0,016% | 0.043% | 0.162% |
| Disintegration time | 8 seconds | 10 seconds | 10 seconds | 10 seconds |

As shown in Table 1, it became clear that the orally disintegrating tablets produced in the examples, which are embodiments of the present invention, have a friability decreased to 1/2 to 1/10 as compared with the orally disintegrating tablets of Comparative Example 1 produced without using dry granulation as in conventional procedures, while the orally disintegrating tablets produced in the examples are excellent in terms of having adequate mechanical strength and disintegration time as compared with those produced by conventional procedures.
Thus, it became clear that an orally disintegrating tablet according to the present invention is the orally disintegrating tablet that hardly cracks or chips during production, packaging, transport, and the like, as compared with those produced by conventional procedures.

### Examination Example 2

Next, the orally disintegrating tablets produced in Example 3 and the orally disintegrating tablets produced in Comparative Example 2 were allowed to stand for 12 hours under the conditions of a temperature of 60°C and a humidity of 75%, and the amount of water absorption per tablet was determined from the amount of increase in tablet weight.

**[Table 2]**

| | Example 3 | Comparative Examples 2 |
|---|---|---|
| Amount of water absorption per tablet per | 0.87mg | 2.63mg |

As shown in Table 2, it became clear that the orally disintegrating tablets produced in Example 3, which is an embodiment of the present invention, has a lower amount of water absorption than the orally disintegrating tablets of Comparative Example 2 produced by dry granulation with crystalline cellulose. That is to say, it became clear that an orally disintegrating tablet obtained according to the present invention has a reduced amount of water absorption in the oral cavity and has superior storage stability, as compared with those of conventional procedures. It is predicted that disintegration of the tablet with such a small amount allows for reduction in discomfort upon ingestion.

Accordingly, it became clear that the present invention can provide an orally disintegrating tablet that disintegrates with a small amount of water, has a rapid disintegration time, and is maintained in a tablet form stably, as compared with those provided by conventional procedures.

### Industrial Applicability

According to the present invention, it has became possible to provide an orally disintegrating tablet that is capable of disintegrating with a small amount of water, having a rapid disintegration time, and being maintained stably in a tablet form.
Thus, it has became possible to provide an orally disintegrating tablet that, in particular, hardly cracks or chips during production, packaging, transport, and the like. Moreover, the orally disintegrating tablet allows for people who have low swallowing ability, such as children and elderly people to be easily and conveniently taken.

## Claims

1. An orally disintegrating tablet produced by dry granulation, the orally disintegrating tablet comprising a medicinal ingredient, silica, and sugar alcohol or/and sugar.

2. The orally disintegrating tablet of claim 1, wherein the medicinal ingredient is water-labile.

3. The orally disintegrating tablet of claim 1, wherein the sugar alcohol is mannitol, erythritol, xylitol, maltitol, sorbitol.

4. The orally disintegrating tablet of claim 1, wherein the sugar is lactose, sucrose, glucose, trehalose.
